# EUROPEAN PATENT APPLICATION

(11) **EP 4 559 399 A1**
(43) Date of publication of application: **28.05.2025**
(21) Application number: 23871377.0
(22) Date of filing: 11.07.2023
(51) Int. Cl.: A61B 6/00, G01T 1/20, G01T 7/00

(54) **INTRAORAL IMAGE CAPTURING DEVICE**

(30) Priority: 28.09.2022 JP 2022154987
(71) Applicant: Hamamatsu Photonics K.K., Hamamatsu-shi, Shizuoka 435-8558 (JP)
(72) Inventor: TSUDA, Yukihiro, Hamamatsu-shi, Shizuoka 435-8558 (JP); MATSUOKA, Kenta, Hamamatsu-shi, Shizuoka 435-8558 (JP); KITAMURA, Shigehiro, Hamamatsu-shi, Shizuoka 435-8558 (JP)
(74) Representative: Hoffmann Eitle
(86) International application number: PCT/JP2023/025612
(87) International publication number: WO 2024/070133

(57) **Abstract**

An intraoral image capturing device includes: a radiation detection unit that detects radiation; a first substrate provided with a communication device capable of wirelessly communicating with an outside; a second substrate provided with a battery and disposed on a side opposite to the radiation detection unit with respect to the first substrate; a connection member that connects the second substrate to the first substrate; and a housing that houses the radiation detection unit, the first substrate, the second substrate, and the connection member. When viewed from the Z direction, the entire outer edge of the second substrate is located inside the outer edge of the first substrate.

## Description

### Technical Field

One aspect of the present disclosure relates to an intraoral image capturing device.

### Background Art

Patent Literature 1 describes an intraoral image capturing device that is disposed in the oral cavity of a patient and captures an image of radiation emitted from outside the oral cavity and transmitted through the patient. In this intraoral image capturing device, a sensor that detects radiation is disposed in a housing, and a light source, which is an element for wireless communication, is provided at a tip of a cable pulled out from the housing. This intraoral image capturing device is used in a state in which the housing is disposed in the oral cavity, the cable is pulled out from the oral cavity, and the light source is located outside the oral cavity.

### Citation List

### Patent Literature

Patent Literature 1: Specification of US Patent No. 7891871

### Summary of Invention

### Technical Problem

The intraoral image capturing device as described above is required to be downsized so that the entire device can be disposed in the oral cavity of a patient, for example. On the other hand, for example, in a case where the configuration, as described above, in which the cable is not pulled out from the housing is adopted in order to dispose the entire device in the oral cavity, the user easily drops the housing by mistake. Therefore, it is required to enhance strength against impact while achieving downsizing.

An object of one aspect of the present disclosure is to provide an intraoral image capturing device capable of enhancing strength against impact while achieving downsizing.

### Solution to Problem

An intraoral image capturing device according to one aspect of the present disclosure is [1] "an intraoral image capturing device comprising: a radiation detection unit that detects radiation; a first substrate provided with a communication device capable of wirelessly communicating with an outside; a second substrate provided with a battery and disposed on a side opposite to the radiation detection unit with respect to the first substrate; a connection member that connects the second substrate to the first substrate; and a housing that houses the radiation detection unit, the first substrate, the second substrate, and the connection member, wherein an entire outer edge of the second substrate is located inside an outer edge of the first substrate when viewed from the thickness direction of the first substrate".

In this intraoral image capturing device, the radiation detection unit that detects radiation, the first substrate provided with a communication device capable of wirelessly communicating with the outside, and the second substrate provided with a battery are housed in a housing. As a result, for example, it is possible to downsize the device so that the entire device can be disposed in the oral cavity of the patient. On the other hand, in a case where the device is downsized, the area for mounting electronic components and the like can be reduced. In this respect, in this intraoral image capturing device, the second substrate is disposed on the side opposite to the radiation detection unit with respect to the first substrate. As a result, for example, the mounting area can be increased as compared with a case where only the first substrate is provided. Furthermore, since the battery is provided on the second substrate, the mounting area can be increased in the first substrate in which the mounting area is likely to be insufficient. Furthermore, when viewed from the thickness direction of the first substrate, the entire outer edge of the second substrate is located inside the outer edge of the first substrate. As a result, the size of the intraoral image capturing device when viewed from the thickness direction of the first substrate can be reduced. Moreover, the second substrate is connected to the first substrate by the connection member, and the battery is provided on the second substrate. As a result, the impact acting on the housing can be prevented from being transmitted to the battery, and the strength against the impact can be enhanced. Therefore, according to this intraoral image capturing device, the strength against impact can be enhanced while achieving downsizing.

The intraoral image capturing device according to one aspect of the present disclosure may be [2] "the intraoral image capturing device according to [1], wherein the second substrate is separated from the first substrate in the thickness direction of the first substrate". In this case, for example, the electronic component and the like can be disposed between the first substrate and the second substrate, and the mounting area can be further increased. Furthermore, since the second substrate is separated from the first substrate, the impact acting on the housing can be further prevented from being transmitted to the battery.

The intraoral image capturing device according to one aspect of the present disclosure may be [3] "the intraoral image capturing device according to claim 2, wherein the first substrate is further provided with an electronic component, and the electronic component is disposed between the first substrate and the second substrate". In this case, the mounting area can be further increased. Furthermore, since the second substrate is disposed between the electronic component and the housing, the electronic component can be protected even when an impact acts on the housing by being bitten by a patient at the time of use, for example.

The intraoral image capturing device according to one aspect of the present disclosure may be [4] "the intraoral image capturing device according to any one of [1] to [3], wherein the connection member has flexibility". In this case, the impact acting on the housing can be further prevented from being transmitted to the battery, and the strength against the impact can be further enhanced.

The intraoral image capturing device according to one aspect of the present disclosure may be [5] "the intraoral image capturing device according to any one of [1] to [4], wherein the connection member is connected to the first substrate or the second substrate via a connector, and is attachable to and detachable from the first substrate or the second substrate". In this case, for example, replacement work of the battery can be facilitated.

The intraoral image capturing device according to one aspect of the present disclosure may be [6] "the intraoral image capturing device according to any one of [1] to [5], wherein the connection member includes a curved portion that is curved so as to protrude in a direction parallel to a main surface of the first substrate". In this case, the impact acting on the housing can be further prevented from being transmitted to the battery, and the strength against the impact can be further enhanced.

The intraoral image capturing device according to one aspect of the present disclosure may be [7] "the intraoral image capturing device according to any one of [1] to [6], wherein the connection member is disposed between the first substrate and the second substrate and supports the second substrate". In this case, the second substrate can be reliably supported, and the strength against an impact can be further enhanced.

The intraoral image capturing device according to one aspect of the present disclosure may be [8] "the intraoral image capturing device according to [7], wherein the connection member is attachable to and detachable from at least one of the first substrate and the second substrate". In this case, for example, replacement work of the battery can be facilitated.

The intraoral image capturing device according to one aspect of the present disclosure may be [9] "the intraoral image capturing device according to any one of [1] to [8], further comprising a cushion member disposed between the first substrate and the housing". In this case, the impact acting on the housing can be absorbed by the cushion member, and the strength against the impact can be further enhanced.

The intraoral image capturing device according to one aspect of the present disclosure may be [10] "the intraoral image capturing device according to [9], wherein the cushion member includes a main surface cushion member disposed between a main surface of the first substrate and the housing". In this case, for example, the impact acting on the housing along the direction perpendicular to the main surface of the first substrate can be suitably absorbed by the main surface cushion member, and the strength against the impact can be further enhanced.

The intraoral image capturing device according to one aspect of the present disclosure may be [11] "the intraoral image capturing device according to [10], wherein the main surface cushion member includes a first cushion member disposed between the second substrate and the housing". In this case, the impact acting on the housing can be absorbed by the first cushion member, and the strength against the impact can be further enhanced.

The intraoral image capturing device according to one aspect of the present disclosure may be [12] "the intraoral image capturing device according to [10] or [11], wherein the main surface cushion member includes a second cushion member disposed between the first substrate and the second substrate". In this case, the impact acting on the housing can be absorbed by the second cushion member, and the strength against the impact can be further enhanced.

The intraoral image capturing device according to one aspect of the present disclosure may be [13] "the intraoral image capturing device according to any one of [10] to [12], wherein the main surface cushion member includes a third cushion member including a portion located inside an outer edge of the first substrate and outside an outer edge of the second substrate when viewed from the thickness direction of the first substrate". In this case, the impact acting on the housing can be absorbed by the third cushion member, and the strength against the impact can be further enhanced.

The intraoral image capturing device according to one aspect of the present disclosure may be [14] "the intraoral image capturing device according to any one of [9] to [13], wherein the cushion member includes a side surface cushion member disposed between a side surface of the first substrate and the housing". In this case, for example, the impact acting on the housing along the direction parallel to the main surface of the first substrate can be suitably absorbed by the side surface cushion member, and the strength against the impact can be further enhanced.

The intraoral image capturing device according to one aspect of the present disclosure may be [15] "the intraoral image capturing device according to any one of [1] to [14], wherein the housing includes a cushion portion formed by forming an inner portion of the housing to be softer than an outer portion of the housing, and the cushion portion is in contact with at least one of the first substrate and the second substrate". In this case, the impact acting on the housing can be absorbed by the cushion portion, and the strength against the impact can be further enhanced.

The intraoral image capturing device according to one aspect of the present disclosure may be [16] "the intraoral image capturing device according to any one of [1] to [15], wherein the size of the battery is 5% or more of the size of the first substrate when viewed from the thickness direction of the first substrate". In this case, the capacity of the battery can be increased. On the other hand, in a case where the battery is large, it becomes difficult to secure an area for mounting other electronic components and the like. In this respect, in this intraoral image capturing device, since the battery is provided on the second substrate, the mounting area can be secured even in such a case.

The intraoral image capturing device according to one aspect of the present disclosure may be [17] "the intraoral image capturing device according to any one of [1] to [16], wherein the battery is provided on a surface of the second substrate, the surface being on a side opposite to the first substrate side". In this case, since the battery is not provided between the first substrate and the second substrate, for example, even if an impact acts on the housing in a case where the electronic component is disposed on a surface of the first substrate, the surface facing the second substrate, the battery and the electronic component do not come into contact with each other, so that damage to the battery and the electronic component can be suppressed.

The intraoral image capturing device according to one aspect of the present disclosure may be [18] "the intraoral image capturing device according to any one of [1] to [16], wherein the battery is provided on a surface of the second substrate, the surface being on a side of the first substrate". In this case, since the second substrate is disposed between the battery and the housing, the battery can be protected even when an impact acts on the housing by being bitten by a patient at the time of use, for example.

The intraoral image capturing device according to one aspect of the present disclosure may be [19] "the intraoral image capturing device according to any one of [1] to [18], wherein the housing includes a first wall portion disposed on a side opposite to the second substrate with respect to the first substrate, a second wall portion disposed on a side opposite to the first substrate with respect to the second substrate, and a side wall portion connected to the first wall portion and the second wall portion, and an outer surface of the wall portion is formed flat over the entire surface. In this case, it is possible to improve patient comfort at the time of use.

The intraoral image capturing device according to one aspect of the present disclosure may be [20] "the intraoral image capturing device according to any one of [1] to [19], wherein the battery is a film-shaped battery". In this case, the size of the housing in the thickness direction of the first substrate can be reduced.

The intraoral image capturing device according to one aspect of the present disclosure may be [21] "the intraoral image capturing device according to any one of [1] to [20], wherein the second substrate is further provided with a battery control unit that controls the battery". In this case, since the battery control unit is provided on the second substrate, it is possible to increase the mounting area of other elements on the first substrate.

The intraoral image capturing device according to one aspect of the present disclosure may be [22] "the intraoral image capturing device according to any one of [1] to [21], wherein at least a part of the communication device is located outside an outer edge of the battery when viewed from the thickness direction of the first substrate". In this case, it is possible to prevent the battery from affecting the operation of the communication device.

The intraoral image capturing device according to one aspect of the present disclosure may be [23] "the intraoral image capturing device according to any one of [1] to [22], wherein the entire communication device is located outside an outer edge of the battery when viewed from the thickness direction of the first substrate". In this case, it is possible to further prevent the battery from affecting the operation of the communication device.

### Advantageous Effects of Invention

According to one aspect of the present disclosure, it is possible to provide an intraoral image capturing device capable of enhancing strength against an impact while achieving downsizing.

### Brief Description of Drawings

FIG. 1 is a perspective view of an intraoral image capturing device according to an embodiment.
FIG. 2 is a plan view of the intraoral image capturing device.
FIG. 3 is a sectional view taken along line III-III of FIG. 2.
FIG. 4 is a view illustrating a first substrate, a second substrate, and a connection member.
FIG. 5 is a cross-sectional view of an intraoral image capturing device according to a first modification.
FIG. 6 is a cross-sectional view of an intraoral image capturing device according to a second modification.
FIG. 7 is a cross-sectional view of an intraoral image capturing device according to a third modification.
FIG. 8 is a cross-sectional view of an intraoral image capturing device according to a fourth modification.
FIG. 9 is a cross-sectional view of an intraoral image capturing device according to a fifth modification.
FIG. 10 is a plan view of an intraoral image capturing device according to a sixth modification.
FIG. 11 is a plan view of an intraoral image capturing device according to a seventh modification.
FIG. 12 is a plan view of an intraoral image capturing device according to an eighth modification.
FIG. 13 is a plan view of an intraoral image capturing device according to a ninth modification.

### Description of Embodiments

Hereinafter, an embodiment of one aspect of the present disclosure will be described in detail with reference to the drawings. The following description will use the same reference numerals for the same or equivalent elements, and repeated descriptions will be omitted.

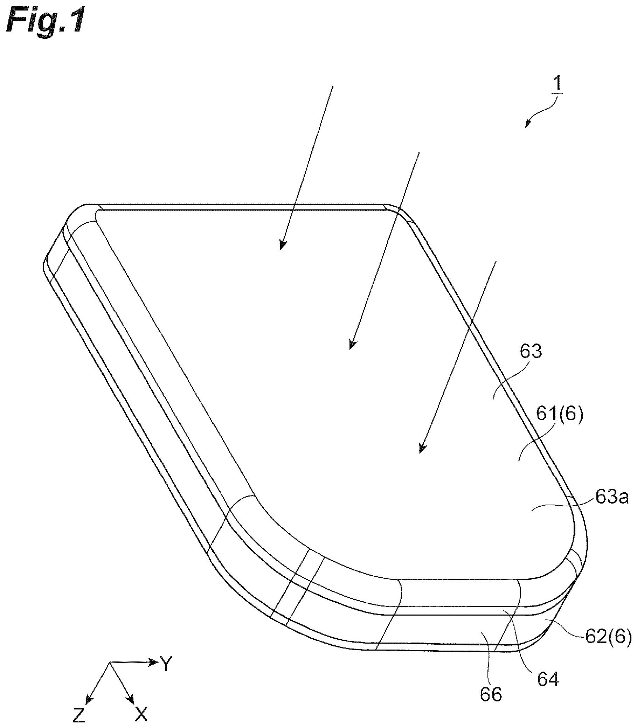

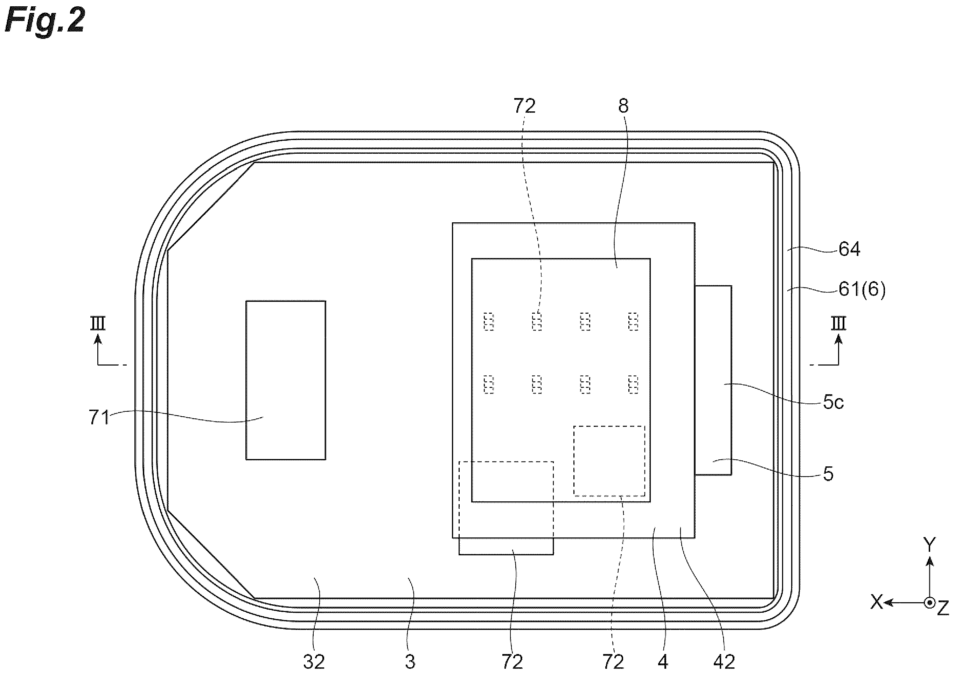

An intraoral image capturing device 1 illustrated in FIGS. 1 to 4 is, for example, a device that is disposed in the oral cavity of a patient and used to capture an image of radiation emitted from the outside of the oral cavity and transmitted through the patient (for example, teeth). That is, the intraoral image capturing device 1 is used in a state where the entire intraoral image capturing device 1 is disposed in the oral cavity. The intraoral image capturing device 1 is a wireless intraoral sensor, and transmits acquired imaging data to the outside of the intraoral image capturing device 1 by wireless communication. The intraoral image capturing device 1 includes a radiation detection unit 2, a first substrate 3, a second substrate 4, a connection member 5, and a housing 6. Hereinafter, an X direction, a Y direction, and a Z direction that are set as illustrated in the drawings will be described. The X direction, the Y direction, and the Z direction are perpendicular to each other. In FIG. 2, illustration of a second cover 62 of the housing 6 to be described later is omitted.

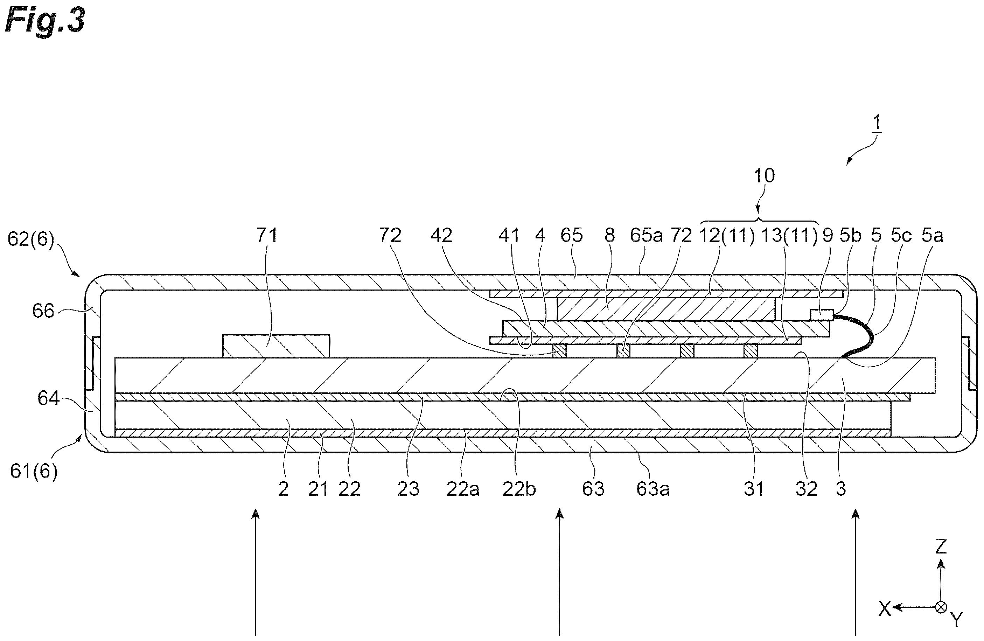

The radiation detection unit 2 includes a scintillator 21, a fiber optical plate (FOP) 22, and a readout substrate 23. The scintillator 21 converts incident radiation (for example, X-rays) into fluorescence. In the intraoral image capturing device 1, as indicated by arrows in FIGS. 1 and 3, radiation transmitted through a first cover 61 of the housing 6 described later is incident on the scintillator 21. The scintillator 21 is formed in a sheet shape, for example, and is disposed over an input surface 22a of the FOP 22. The FOP 22 is an optical device configured by bundling a plurality of (a large number of) optical fibers, and includes the input surface 22a and an output surface 22b on a side opposite to the input surface 22a. The input surface 22a and the output surface 22b are flat surfaces perpendicular to the Z direction in this example. The input surface 22a is constituted by surfaces of one end of a plurality of optical fibers, and the output surface 22b is constituted by surfaces of the other end of the plurality of optical fibers. The FOP 22 is disposed on the readout substrate 23 such that the output surface 22b faces the readout substrate 23. In the FOP 22, light incident on the input surface 22a propagates in the optical fiber and is output from the output surface 22b.

The readout substrate 23 is, for example, a circuit board (an image sensor) in which a light receiving unit (a light receiving element) is built. In the radiation detection unit 2, fluorescence generated in the scintillator 21 is guided by the FOP 22, is incident on the light receiving unit of the readout substrate 23, and is detected by the readout substrate 23. Note that, in this example, the radiation is an X-ray, but the radiation is not limited to an X-ray, and may be a γ-ray, for example. The radiation detection unit 2 is not limited to the above configuration as long as it can detect radiation. For example, the FOP 22 may be omitted, and the scintillator 21 may be directly disposed on the readout substrate 23. Alternatively, instead of the scintillator 21 and the FOP 22, a direct conversion substrate that directly converts incident radiation into an electric signal may be provided. In this case, the readout substrate 23 may not include the light receiving unit.

The first substrate 3 includes a first main surface 31 and a second main surface 32 opposite to the first main surface 31. In this example, the first main surface 31 and the second main surface 32 are flat surfaces perpendicular to the Z direction (the thickness direction of the first substrate 3). The first substrate 3 is formed in a substantially rectangular shape having long sides parallel to the X direction when viewed from the Z direction. In this example, a pair of corners located on one side in the X direction is chamfered (C-chamfered), and the first substrate 3 is formed in a hexagonal shape.

The radiation detection unit 2 described above is disposed on the first main surface 31. The scintillator 21, the FOP 22, and the readout substrate 23 described above may be formed in a hexagonal shape corresponding to the first substrate 3. The radiation detection unit 2 is formed to be smaller than the first substrate 3 when viewed from the Z direction, and is disposed so as not to protrude outward from the first substrate 3 when viewed from the Z direction.

A communication device 71 and a plurality of electronic components 72 are provided on the second main surface 32. The communication device 71 is configured to be able to wirelessly communicate with the outside of the intraoral image capturing device 1. For example, the communication device 71 receives a control signal for controlling the intraoral image capturing device 1 from an external controller configured by a computer. Furthermore, the communication device 71 transmits the imaging data acquired by the radiation detection unit 2 to the external controller. The wireless communication (wireless link) means may be Bluetooth (registered trademark), Wi-Fi, or the like, or may be other communication means. The plurality of electronic components 72 include, for example, a sensor control IC (a field-programmable gate array (FPGA)), a power supply control IC (a battery control unit), a memory IC, and the like.

The second substrate 4 is disposed on the side (the upper side in FIG. 3) opposite to the radiation detection unit 2 with respect to the first substrate 3. The second substrate 4 includes a third main surface 41 and a fourth main surface 42. In this example, the third main surface 41 and the fourth main surface 42 are flat surfaces perpendicular to the Z direction (the thickness direction of the first substrate 3). The third main surface 41 faces the second main surface 32 of the first substrate 3. The second substrate 4 is formed in a substantially rectangular shape having long sides parallel to the Y direction when viewed from the Z direction.

A battery 8 is provided on the fourth main surface 42. The battery 8 is a power source of the intraoral image capturing device 1, and supplies power to, for example, the communication device 71 and the electronic component 72. In this example, the battery 8 is a film-shaped battery, and is formed in, for example, a film shape and has a thickness of 1 mm or less. The battery 8 can be charged by, for example, wireless charging or wired charging.

The second substrate 4 is electrically and physically connected to the first substrate 3 by the connection member 5. The connection member 5 is formed of, for example, a rectangular sheet-shaped flexible printed circuit (FPC), and has flexibility. As illustrated in FIG. 3, the connection member 5 extends so as to be bridged between the first substrate 3 and the second substrate 4. The connection member 5 has a curved portion 5c that is curved so as to protrude in a direction parallel to the first main surface 31 of the first substrate 3. When viewed from the Y direction (one direction perpendicular to the Z direction), the curved portion 5c is curved so as to protrude outward in the X direction (the Z direction and the direction perpendicular to the one direction).

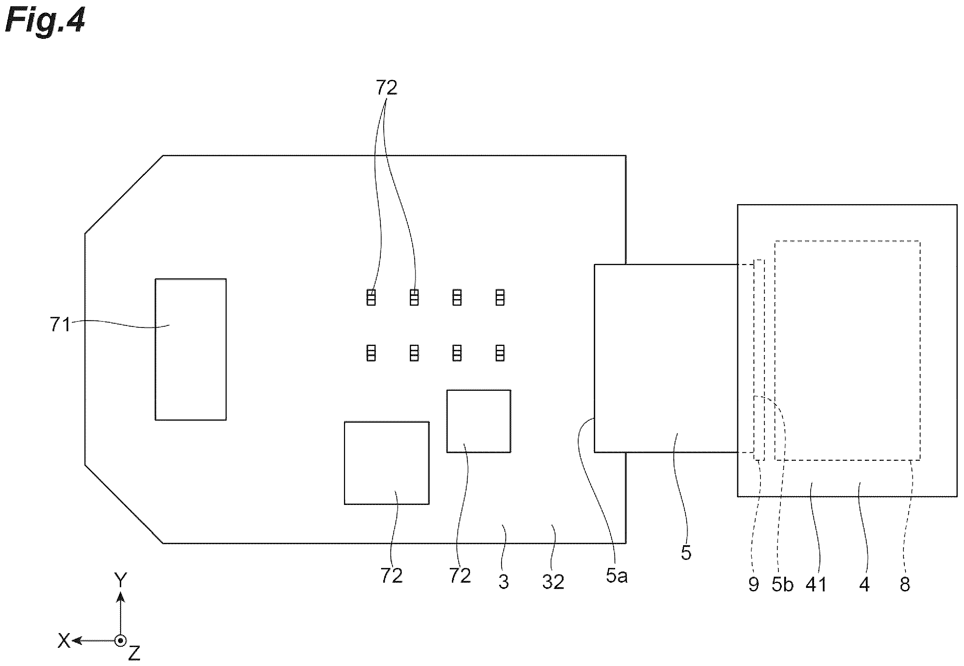

One edge portion 5a of the connection member 5 is connected to the first substrate 3 by, for example, solder. The other edge portion 5b of the connection member 5 is connected to the second substrate 4 via a connector 9. The other edge portion 5b is attachable to and detachable from the connector 9, whereby the connection member 5 is attachable to and detachable from the second substrate 4. The connector 9 is provided, for example, on the fourth main surface 42 of the second substrate 4 along one edge portion in the X direction. Since the connection member 5 has flexibility, the connection member 5 can be deformed into a curved (folded back) state as illustrated in FIG. 3 or a state of extending in a planar shape as illustrated in FIG. 4. The connection member 5 is in a curved state as illustrated in FIG. 3 when the intraoral image capturing device 1 is used.

The housing 6 is formed in a flat box shape, and houses the radiation detection unit 2, the first substrate 3, the second substrate 4, and the connection member 5. The housing 6 includes the first cover 61 constituting a portion on one side in the Z direction and a second cover 62 constituting a portion on the other side in the Z direction. The first cover 61 includes a bottom wall portion 63 (a first wall portion) extending perpendicularly to the Z direction, and a side wall portion 64 provided to surround the bottom wall portion 63. The second cover 62 includes a bottom wall portion 65 (a second wall portion) extending perpendicularly to the Z direction, and a side wall portion 66 provided to surround the bottom wall portion 65. The housing 6 is formed of, for example, nylon or ABS resin.

The first cover 61 and the second cover 62 are engaged with each other in the side wall portions 64 and 66. In a state where the first cover 61 and the second cover 62 are engaged with each other, the side wall portions 64 and 66 constitute one side wall portion connected to the bottom wall portion 63 and the bottom wall portion 65. The first cover 61 and the second cover 62 have, for example, symmetrical shapes with respect to the Z direction except for the engagement portion. In this example, each of an outer surface 63a of the bottom wall portion 63 and an outer surface 65a of the bottom wall portion 65 is formed flat over the entire surface. The bottom wall portions 63 and 65 are formed in a substantially rectangular shape having long sides parallel to the X direction when viewed from the Z direction. In this example, a pair of corners located on one side in the X direction is curved in an R shape so that the housing 6 has a shape corresponding to the shape of the first substrate 3.

The disposition of each member in the housing 6 will be further described. The intraoral image capturing device 1 further includes a cushion member 10 disposed between the first substrate 3 and the housing 6. The cushion member 10 includes a first cushion member 12 and a second cushion member 13. The first cushion member 12 and the second cushion member 13 are main surface cushion members 11 disposed between the second main surface 32 of the first substrate 3 and the housing 6. In the intraoral image capturing device 1, the radiation detection unit 2, the first substrate 3, the second substrate 4, and the like are disposed without a gap in the Z direction in the housing 6 with the first cushion member 12 and the second cushion member 13 interposed therebetween, whereby the radiation detection unit 2, the first substrate 3, the second substrate 4, and the like are positioned and held (fixed) in the housing 6. The first cushion member 12 and the second cushion member 13 are formed in a sheet shape, for example. The first cushion member 12 and the second cushion member 13 may be formed of, for example, silicone rubber, or may be formed by curing an injected resin. The materials of the first cushion member 12 and the second cushion member 13 may be insulating materials as described above, or may be conductive materials. The material of the first cushion member 12 and the second cushion member 13 may be a material having a Young's modulus smaller than that of the material of the housing 6.

The radiation detection unit 2 and the first substrate 3 are disposed on the first cover 61 side so that the scintillator 21 is in contact with the bottom wall portion 63 of the first cover 61. The second substrate 4 is disposed so as to be away from the first substrate 3 in the Z direction, and in this example, a gap is formed between the third main surface 41 of the second substrate 4 and the second main surface 32 of the first substrate 3. The plurality of electronic components 72 are disposed in this gap. That is, the plurality of electronic components 72 is disposed between the first substrate 3 and the second substrate 4.

The second cushion member 13 is disposed between the first substrate 3 and the second substrate 4. For example, the second cushion member 13 is disposed between the second substrate 4 and the plurality of electronic components 72. The second cushion member 13 is interposed between the third main surface 41 of the second substrate 4 and the plurality of electronic components 72, and is in contact with the third main surface 41 and the plurality of electronic components 72. The second cushion member 13 may exist not only between the third main surface 41 and the electronic component 72 but also in a gap between the plurality of electronic components 72. The second cushion member 13 existing in the gap may be, for example, a portion pushed out between the electronic components 72 when the second cushion member 13 formed in a sheet shape is pressed, or may be a portion made of resin injected and cured. The first cushion member 12 is disposed between the second substrate 4 and the housing 6. For example, the first cushion member 12 is disposed between the second substrate 4 and the bottom wall portion 65 of the second cover 62 of the housing 6. The first cushion member 12 is interposed between the battery 8 provided on the fourth main surface 42 of the second substrate 4 and the bottom wall portion 65, and is in contact with the battery 8 and the bottom wall portion 65. Note that, in the first cushion member 12, the first cushion member 12 may have a portion pushed out to the side surface side of the battery 8. The portion of the first cushion member 12 may be in contact with the fourth main surface 42 of the second substrate 4.

With the above disposition, the radiation detection unit 2, the first substrate 3, the second substrate 4, and the like are fixed in the housing 6. In the fixed state, the bottom wall portion 63 is located on the side (the lower side in FIG. 3) opposite to the second substrate 4 with respect to the first substrate 3, and the bottom wall portion 65 is located on the side (the upper side in FIG. 3) opposite to the first substrate 3 with respect to the second substrate 4.

With reference to FIGS. 2 and 3, the positional relationship of each member when viewed from the Z direction will be described. The entire outer edge of the second substrate 4 is located inside the outer edge of the first substrate 3. That is, the second substrate 4 is formed to be smaller than the first substrate 3 when viewed from the Z direction, and is disposed so as not to protrude outward from the first substrate 3 when viewed from the Z direction. The battery 8 is disposed at the central portion of the fourth main surface 42 of the second substrate 4. The size (area) of the battery 8 is 5% or more of the size (area) of the first substrate 3, and in this example, is 30% or more of the size of the first substrate 3. In this example, the communication device 71 is disposed so as to be entirely located outside the outer edge of the battery 8. That is, the communication device 71 does not overlap the battery 8 in the Z direction. On the other hand, in some of the plurality of electronic components 72, some or all thereof are disposed so as to overlap the battery 8 in the Z direction. Note that, the battery 8 may be larger than the second substrate 4 when viewed from the Z direction.

### [Functions and Effects]

In the intraoral image capturing device 1, the radiation detection unit 2 that detects radiation, the first substrate 3 provided with the communication device71 capable of wirelessly communicating with the outside, and the second substrate 4 provided with the battery 8 are housed in the housing 6. As a result, for example, it is possible to downsize the device so that the entire device can be disposed in the oral cavity of the patient. On the other hand, in a case where the device is downsized, the area for mounting the communication device 71, the electronic component 72, and the like can be reduced. In this respect, in this intraoral image capturing device 1, the second substrate 4 is disposed on the side opposite to the radiation detection unit 2 with respect to the first substrate 3. As a result, for example, the mounting area can be increased as compared with a case where only the first substrate 3 is provided. Furthermore, since the battery 8 is provided on the second substrate 4, the mounting area can be increased in the first substrate 3 in which the mounting area is likely to be insufficient. Furthermore, when viewed from the Z direction (the thickness direction of the first substrate 3), the entire outer edge of the second substrate 4 is located inside the outer edge of the first substrate 3. As a result, the size of the intraoral image capturing device 1 when viewed from the Z direction can be reduced. Moreover, the second substrate 4 is connected to the first substrate 3 by the connection member 5, and the battery 8 is provided on the second substrate 4. As a result, the impact acting on the housing 6 can be prevented from being transmitted to the battery 8, and the strength against the impact can be enhanced. Therefore, according to the intraoral image capturing device 1, the strength against impact can be enhanced while achieving downsizing.

Furthermore, in the intraoral image capturing device 1, it is possible to mount electronic components and the like on both main surfaces (the third main surface 41 and the fourth main surface 42) of the second substrate 4, and this also makes it possible to increase the mounting area. Furthermore, since the size of the second substrate 4 when viewed from the Z direction only needs to be smaller than the size of the first substrate 3, the size of the battery 8 when viewed from the Z direction can be increased to the size of the first substrate 3. That is, since the size of the battery 8 is not limited by the mounting area of the first substrate 3, it is possible to adopt the battery 8 having a larger area. Furthermore, since the heat generated in the battery 8 is transferred to the first substrate 3 via the second substrate 4, it is possible to prevent the heat from being transferred to the radiation detection unit 2 and the first substrate 3 as compared with, for example, a case where the battery 8 is provided on the first substrate 3.

The connection member 5 electrically and physically connects the second substrate 4 to the first substrate 3. As a result, the electrical and physical connection between the first substrate 3 and the second substrate 4 can be realized by the connection member 5.

The second substrate 4 is separated from the first substrate 3 in the Z direction. As a result, the electronic component 72 and the like can be disposed between the first substrate 3 and the second substrate 4, and the mounting area can be further increased. Furthermore, since the second substrate 4 is separated from the first substrate 3, the impact acting on the housing 6 can be further prevented from being transmitted to the battery 8.

The plurality of electronic components 72 are disposed between the first substrate 3 and the second substrate 4. As a result, the mounting area can be further increased. Furthermore, since the second substrate 4 is disposed between the electronic component 72 and the housing 6, the electronic component 72 can be protected even when an impact acts on the housing 6 by being bitten by a patient at the time of use, for example.

The connection member 5 has flexibility. As a result, the impact acting on the housing 6 can be further prevented from being transmitted to the battery 8, and the strength against the impact can be further enhanced. That is, since the second substrate 4 is connected to the first substrate 3 by the connection member 5 having flexibility, the second substrate 4 is not completely fixed and can be moved to some extent, so that the strength against the impact can be further enhanced.

The connection member 5 is connected to the second substrate 4 via the connector 9, and is attachable to and detachable from the second substrate 4. As a result, for example, replacement work of the battery 8 can be facilitated.

The connection member 5 has a curved portion 5c that is curved so as to protrude in a direction parallel to the second main surface 32 of the first substrate 3. As a result, the impact acting on the housing 6 can be further prevented from being transmitted to the battery 8, and the strength against the impact can be further enhanced.

The connection member 5 extends so as to be bridged between the first substrate 3 and the second substrate 4. As a result, the impact acting on the housing 6 can be further prevented from being transmitted to the battery 8, and the strength against the impact can be further enhanced.

The cushion member 10 is disposed between the first substrate 3 and the housing 6. As a result, the impact acting on the housing 6 can be absorbed by the cushion member 10, and the strength against the impact can be further enhanced.

Here, in the conventional wired intraoral image capturing device having the cable pulled out from the housing, the substrate can be fixed and positioned inside the housing by using the cable. On the other hand, in the intraoral image capturing device 1 of the embodiment, such a cable is not provided, and thus it is necessary to fix and position the substrate by using another means. In this respect, in the intraoral image capturing device 1, the substrate (the first substrate 3 or the like) can be fixed and positioned by using the cushion member 10 disposed between the first substrate 3 and the housing 6.

The main surface cushion member 11 is disposed between the second main surface 32 of the first substrate 3 and the housing 6. As a result, for example, the impact acting on the housing 6 along the direction perpendicular to the second main surface 32 of the first substrate 3 can be suitably absorbed by the main surface cushion member 11, and the strength against the impact can be further enhanced.

The first cushion member 12 is disposed between the second substrate 4 and the housing 6. As a result, the impact acting on the housing 6 can be absorbed by the first cushion member 12, and the strength against the impact can be further enhanced.

The second cushion member 13 is disposed between the first substrate 3 and the second substrate 4. As a result, the impact acting on the housing 6 can be absorbed by the second cushion member 13, and the strength against the impact can be further enhanced.

When viewed from the Z direction, the size of the battery 8 is 5% or more of the size of the first substrate 3. As a result, the capacity of the battery 8 can be increased. On the other hand, in a case where the battery 8 is large, it becomes difficult to secure an area for mounting other electronic components and the like. In this respect, in the intraoral image capturing device 1, since the battery 8 is provided on the second substrate 4, the mounting area can be secured even in such a case.

The battery 8 is provided on a surface (the fourth main surface 42) of the second substrate 4, the surface being on a side opposite to the first substrate 3 side. As a result, since the battery 8 is not provided between the first substrate 3 and the second substrate 4, for example, even if an impact acts on the housing 6 in a case where the electronic component 72 is disposed on a surface (the second main surface 32) of the first substrate 3, the surface facing the second substrate 4, the battery 8 and the electronic component 72 do not come into contact with each other, so that damage to the battery 8 and the electronic component 72 can be suppressed.

The outer surface 65a of the bottom wall portion 65 of the housing 6 is formed flat over the entire surface. This makes it possible to improve patient comfort at the time of use. Furthermore, when the outer surface 65a of the bottom wall portion 65 is formed flat over the entire surface, for example, when the battery 8 is charged by wireless charging, charging efficiency can be secured. That is, in a case where the outer surface 65a of the bottom wall portion 65 is not flat over the entire surface, the intraoral image capturing device 1 is inclined with respect to a power transmitting coil, and there is a possibility that axial deviation occurs between a power receiving coil in the housing 6 and the power transmitting coil of the wireless charging device. In this case, since the power receiving coil and the power transmitting coil are not parallel to each other, the charging efficiency decreases. In this respect, in the present embodiment, since the outer surface 65a of the bottom wall portion 65 is formed flat over the entire surface, such a decrease in the charging efficiency can be suppressed. Furthermore, since the bottom wall portion 65 is flat over the entire surface, the number of windings of the power receiving coil in the housing 6 can be increased.

The battery 8 is a film-shaped battery. As a result, the size of the housing 6 in the Z direction can be reduced.

When viewed from the Z direction, the entire communication device 71 is located outside the outer edge of the battery 8. This can prevent the battery 8 from affecting the operation of the communication device 71 (for example, affecting the antenna of the communication device 71).

### [First Modification]

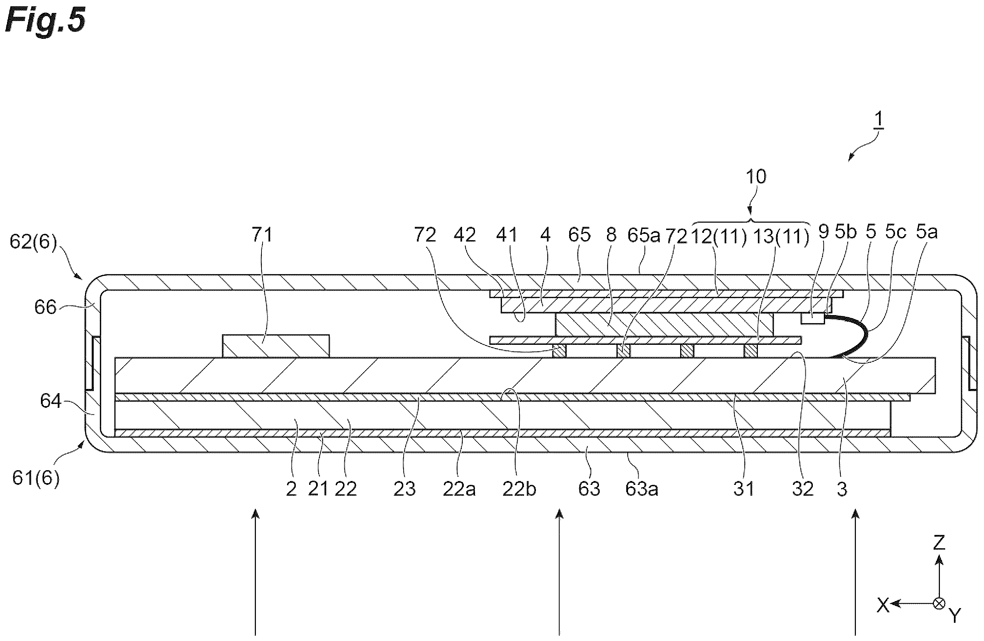

In the first modification illustrated in FIG. 5, the battery 8 is provided on the third main surface 41 (the surface on the first substrate 3 side) of the second substrate 4. The second cushion member 13 is disposed between the battery 8 and the plurality of electronic components 72. The connector 9 is provided on the third main surface 41 of the second substrate 4.

According to such a first modification as well, similarly to the above-described embodiment, the strength against impact can be enhanced while achieving downsizing. Furthermore, the battery 8 is provided on the third main surface 41 of the second substrate 4. As a result, since the second substrate 4 is disposed between the battery 8 and the housing 6, the battery 8 can be protected even when an impact acts on the housing 6 by being bitten by a patient at the time of use, for example.

### [Second Modification]

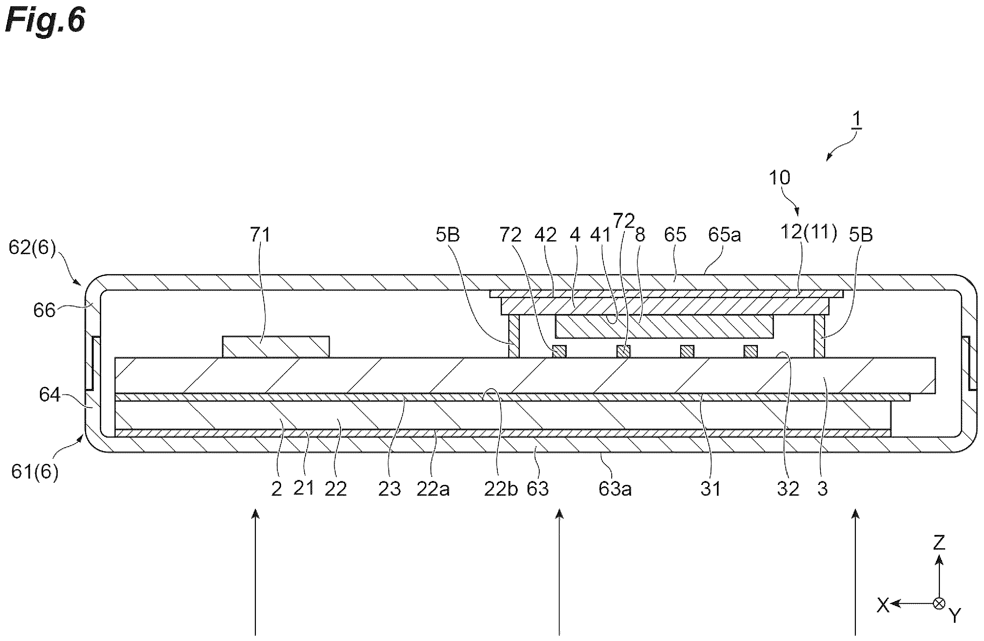

The second modification illustrated in FIG. 6 has the same configuration as that of the first modification except for the following points. In the second modification, the first substrate 3 and the second substrate 4 are connected by a plurality of connection members 5B. Each connection member 5B is, for example, a connector and does not have flexibility. The plurality of connection members 5B are disposed between the first substrate 3 and the second substrate 4 and support the second substrate 4. Each connection member 5B is provided so as to extend along the Z direction. The connection member 5B is attachable to and detachable from at least one of the first substrate 3 and the second substrate 4. Furthermore, in the second modification, the second cushion member 13 is not provided.

According to such a second modification as well, similarly to the above-described embodiment, the strength against impact can be enhanced while achieving downsizing. Furthermore, since the connection member 5B is disposed between the first substrate 3 and the second substrate 4 to support the second substrate 4, the second substrate 4 can be reliably supported, and the strength against impact can be further enhanced. Furthermore, since the connection member 5B is attachable to and detachable from at least one of the first substrate 3 and the second substrate 4, for example, replacement work of the battery 8 can be facilitated.

In the above embodiment, the connection member 5 has flexibility. In this case, since the second substrate 4 can come into contact with the electronic component 72 provided on the second main surface 32 of the first substrate 3 by its own weight, the second cushion member 13 is provided between the second substrate 4 and the first substrate 3. On the other hand, in the second modification, since the second substrate 4 is supported by the connection member 5B, the second substrate 4 does not come into contact with the electronic component 72 as in the case of the above embodiment. Therefore, in the second modification, it is possible to protect the electronic component 72 and the second substrate 4 without including the second cushion member 13.

### [Third Modification]

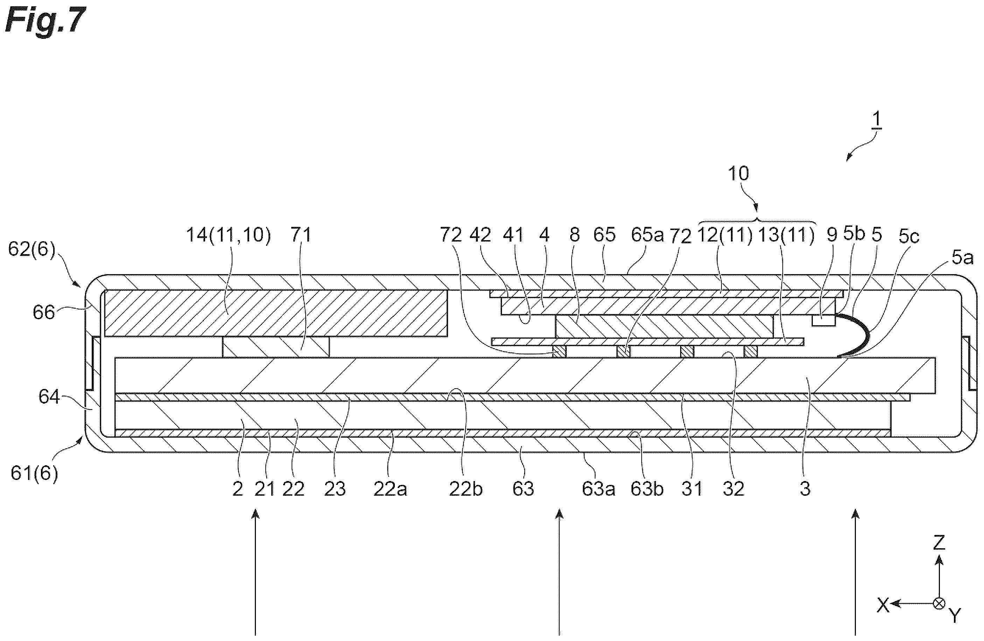

The third modification illustrated in FIG. 7 has the same configuration as that of the first modification except for the following points. In the third modification, the main surface cushion member 11 further includes a third cushion member 14 disposed between the first substrate 3 and the housing 6. The third cushion member 14 has a portion located inside the outer edge of the first substrate 3 and outside the outer edge of the second substrate 4 when viewed from the Z direction. In this example, the third cushion member 14 further includes, in addition to the portion, a portion located outside the outer edge of the first substrate 3 when viewed from the thickness direction of the first substrate 3. The third cushion member 14 may be formed of, for example, silicone rubber, or may be formed by curing an injected resin. In this example, the third cushion member 14 is disposed between the second main surface 32 of the first substrate 3 and the bottom wall portion 65 of the second cover 62. The third cushion member 14 is interposed between the communication device 71 and the bottom wall portion 65, and is in contact with the communication device 71 and the bottom wall portion 65.

According to such a third modification as well, similarly to the above-described embodiment, the strength against impact can be enhanced while achieving downsizing. Furthermore, since the third cushion member 14 is disposed between the first substrate 3 and the housing 6, the impact acting on the housing 6 can be absorbed by the third cushion member 14, and strength against the impact can be further enhanced. Furthermore, since the third cushion member 14 is disposed between the second main surface 32 of the first substrate 3 and the housing 6, for example, the impact acting on the housing 6 along the Z direction (the direction perpendicular to the second main surface 32) can be suitably absorbed by the third cushion member 14, and the strength against the impact can be further enhanced.

Note that, in the intraoral image capturing device 1, the cable is not pulled out from the housing 6 unlike the related art, and the entire device is downsized so as to be disposed in the oral cavity of the patient. On the other hand, in a case where the entire device is downsized as described above, since the user easily accidentally drops the housing, the cushion member is disposed in the housing 6 to enhance the strength against the impact. In this respect, in the intraoral image capturing device 1, since the second substrate 4 is provided and the battery 8 is provided on the second substrate 4, a space for disposing the third cushion member 14 on the first substrate 3 can be suitably secured.

### [Fourth Modification]

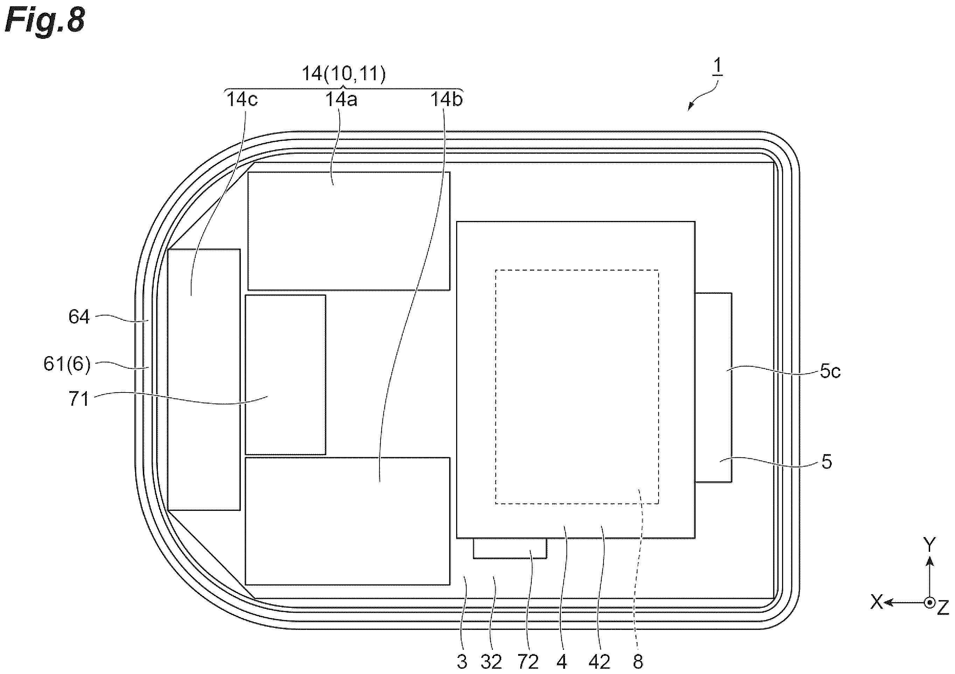

The fourth modification illustrated in FIG. 8 has the same configuration as that of the third modification except for the following points. In the fourth modification, the third cushion member 14 is disposed not between the communication device 71 and the bottom wall portion 65 of the second cover 62 but between the second main surface 32 of the first substrate 3 and the bottom wall portion 65. That is, the third cushion member 14 is disposed so as not to overlap the communication device 71 in the Z direction. Furthermore, in the fourth modification, the third cushion member 14 includes a plurality of (three in this example) cushion members 14a, 14b, and 14c. The cushion members 14a and 14b are disposed on one side and the other side of the communication device 71 in the Y direction, respectively, and the cushion member 14c is disposed on one side of the communication device 71 in the X direction. The entirety of cushion members 14a, 14b, and 14c are located inside the outer edge of the first substrate 3 and outside the outer edge of the second substrate 4 when viewed from the Z direction. The cushion members 14a, 14b, and 14c are interposed between the second main surface 32 of the first substrate 3 and the bottom wall portion 65, and are in contact with the second main surface 32 and the bottom wall portion 65.

According to such a fourth modification as well, similarly to the above-described embodiment, the strength against impact can be enhanced while achieving downsizing. Furthermore, since the third cushion member 14 is disposed between the second main surface 32 of the first substrate 3 and the housing 6, for example, the impact acting on the housing 6 along the Z direction can be suitably absorbed by the third cushion member 14, and the strength against the impact can be further enhanced.

### [Fifth Modification]

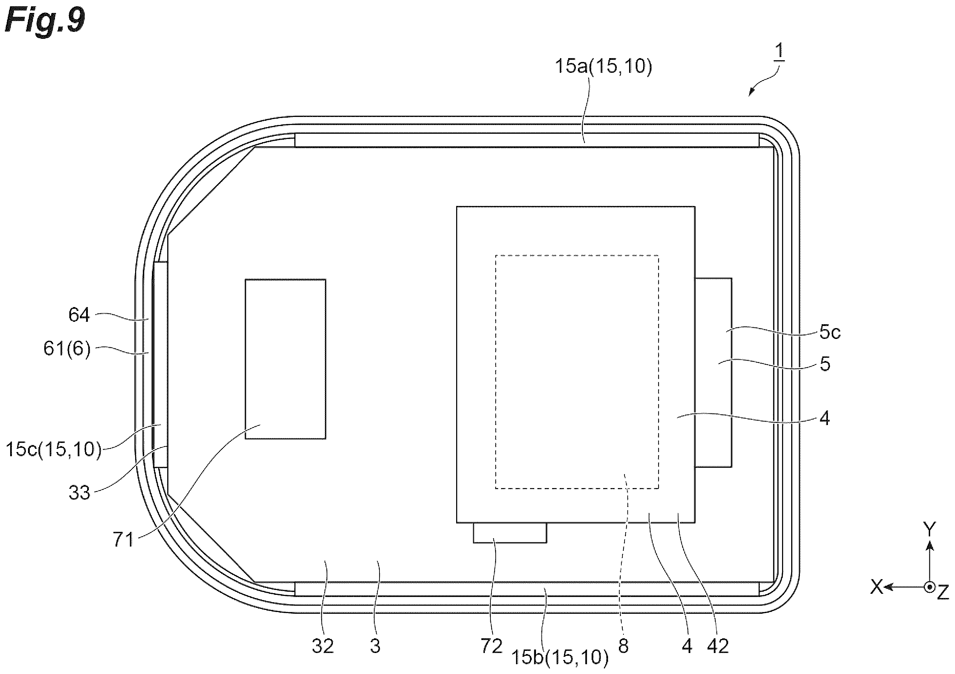

The fifth modification illustrated in FIG. 9 has the same configuration as that of the third modification except for the following points. In the fifth modification, the cushion member 10 includes a side surface cushion member 15 disposed between a side surface 33 of the first substrate 3 and the side wall (the side wall portions 64, 66) of the housing 6. More specifically, the side surface cushion member 15 includes a plurality of (three in this example) cushion members 15a, 15b, and 15c. The cushion members 15a and 15b are disposed on one side and the other side of the first substrate 3 in the Y direction, respectively, and the cushion member 15c is disposed on one side of the first substrate 3 in the X direction. The cushion members 15a, 15b, and 15c are interposed between the side surface 33 of the first substrate 3 and the side wall portions 64 and 66, and are in contact with the side surface 33 and the side wall portions 64 and 66.

According to such a fifth modification as well, similarly to the above-described embodiment, the strength against impact can be enhanced while achieving downsizing. Furthermore, since the side surface cushion member 15 is disposed between the side surface 33 of the first substrate 3 and the housing 6, for example, the impact acting on the housing 6 along a direction parallel to the second main surface 32 can be suitably absorbed by the side surface cushion member 15, and the strength against the impact can be further enhanced. Note that, in the fifth modification, similarly to the fourth modification, the side surface cushion member 15 may also be disposed between the second main surface 32 of the first substrate 3 and the bottom wall portion 65 of the second cover 62.

### [Sixth Modification]

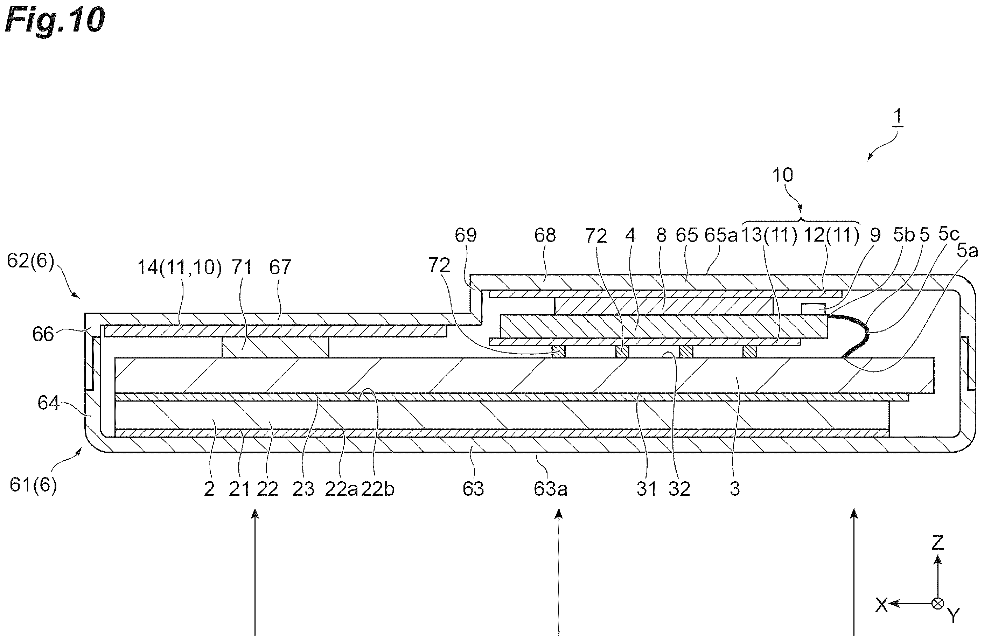

The sixth modification illustrated in FIG. 10 has the same configuration as that of the above embodiment except for the following points. In the sixth modification, the bottom wall portion 65 of the second cover 62 includes a first portion 67 and a second portion 68. The first portion 67 is located on one side in the X direction with respect to the second portion 68. The distance between the first portion 67 and the second main surface 32 of the first substrate 3 in the Z direction is shorter than the distance between the second portion 68 and the second main surface 32 in the Z direction, whereby a step portion 69 is formed between the first portion 67 and the second portion 68. The step portion 69 extends along the Y direction. The third cushion member 14 is disposed between the first substrate 3 and the housing 6. In this example, the third cushion member 14 is disposed between the second main surface 32 of the first substrate 3 and the first portion 67 of the bottom wall portion 65. The third cushion member 14 is interposed between the communication device 71 and the first portion 67, and is in contact with the communication device 71 and the first portion 67. According to such a sixth modification as well, similarly to the above-described embodiment, the strength against impact can be enhanced while achieving downsizing.

Furthermore, in the sixth modification, the distance between the first portion 67 and the second main surface 32 of the first substrate is shorter than the distance between the bottom wall portion 65 and the second main surface 32 of the first substrate in the above embodiment. As a result, the first substrate 3 can be fixed by the third cushion member 14 that is small (less) as compared with the above embodiment. Moreover, in the sixth modification, the second substrate 4 is provided so as to overlap the first portion 67 and the step portion 69 when viewed from the X direction. As a result, the movement of the second substrate 4 to the other side in the X direction is prevented by the first portion 67 and the step portion 69. As a result, the load on the connection member 5 due to the movement of the second substrate 4 can be reduced.

### [Seventh Modification]

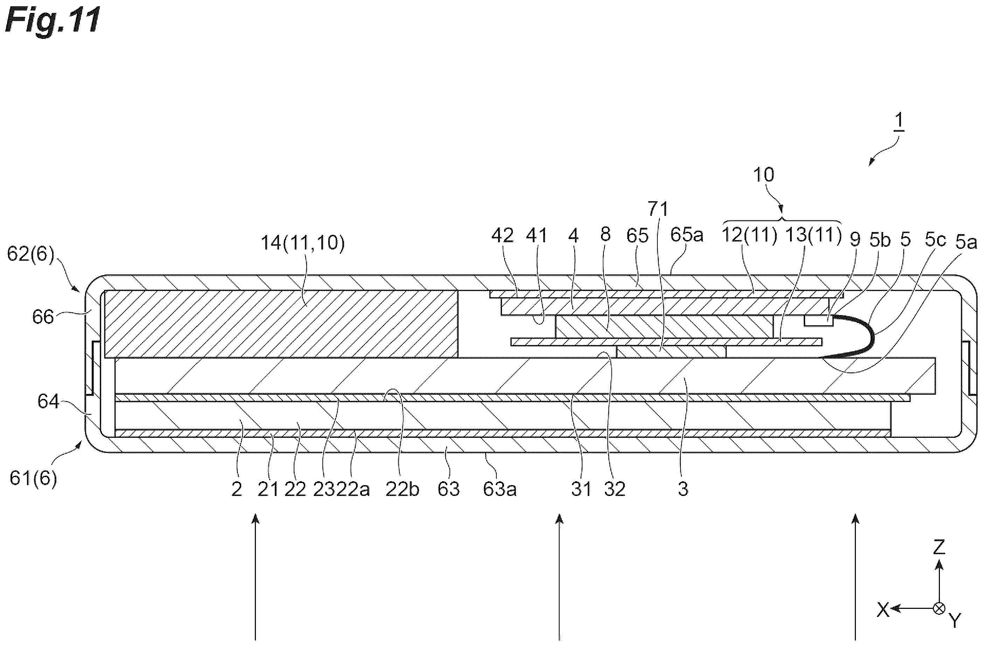

The seventh modification illustrated in FIG. 11 has the same configuration as that of the above third modification except for the following points. In the seventh modification, the communication device 71 is disposed so as to be entirely located inside the outer edge of the battery 8 when viewed from the Z direction. That is, in the seventh modification, the entire communication device 71 overlaps the battery 8 in the Z direction. Furthermore, the third cushion member 14 is interposed between the second main surface 32 of the first substrate 3 and the bottom wall portion 65, and are in contact with the second main surface 32 and the bottom wall portion 65. According to such a sixth modification as well, similarly to the above-described embodiment, the strength against impact can be enhanced while achieving downsizing.

### [Eighth Modification]

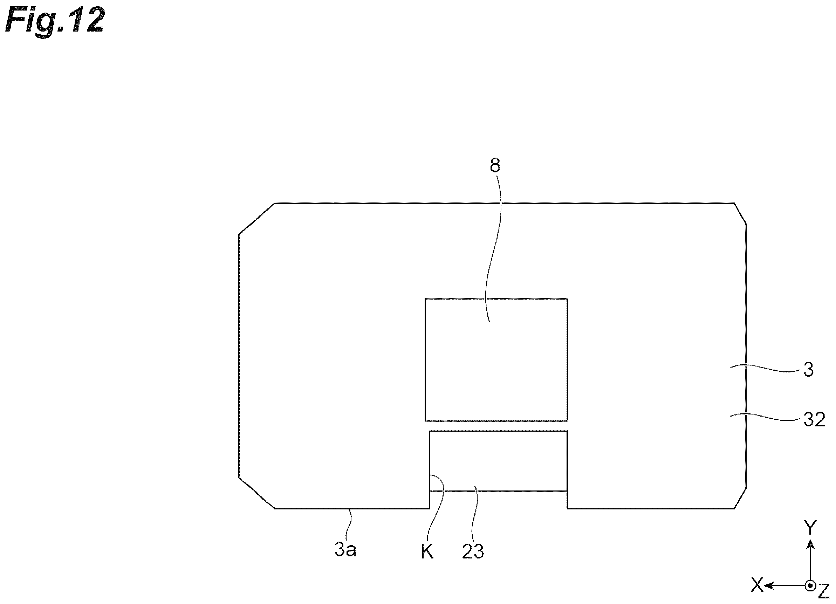

FIG. 12 illustrates an eighth modification. In the eighth modification, the second substrate 4 is not provided, and the battery 8 is provided on the second main surface 32 of the first substrate 3. In FIG. 12, illustration of the housing 6 is omitted. A notch portion K is formed in the first substrate 3. The notch portion K is formed in the long side 3a of the first substrate 3, and extends from a long side 3a toward the battery 8 disposed in the central portion of the first substrate 3. The notch portion K is formed in a rectangular shape having long sides parallel to the X direction when viewed from the Z direction. The notch portion K is adjacent to the battery 8 in the Y direction when viewed from the Z direction. In the eighth modification, a part of the readout substrate 23 is exposed from the notch portion K. That is, the intraoral image capturing device of the eighth modification is "an intraoral image capturing device 1 including: the radiation detection unit 2 that detects radiation; the first substrate 3 on which the communication device 71 capable of wirelessly communicating with the outside and the battery 8 are provided; and the housing 6 that houses the radiation detection unit 2 and the first substrate 3, in which the notch portion K is formed on one side of the first substrate 3".

### [Ninth Modification]

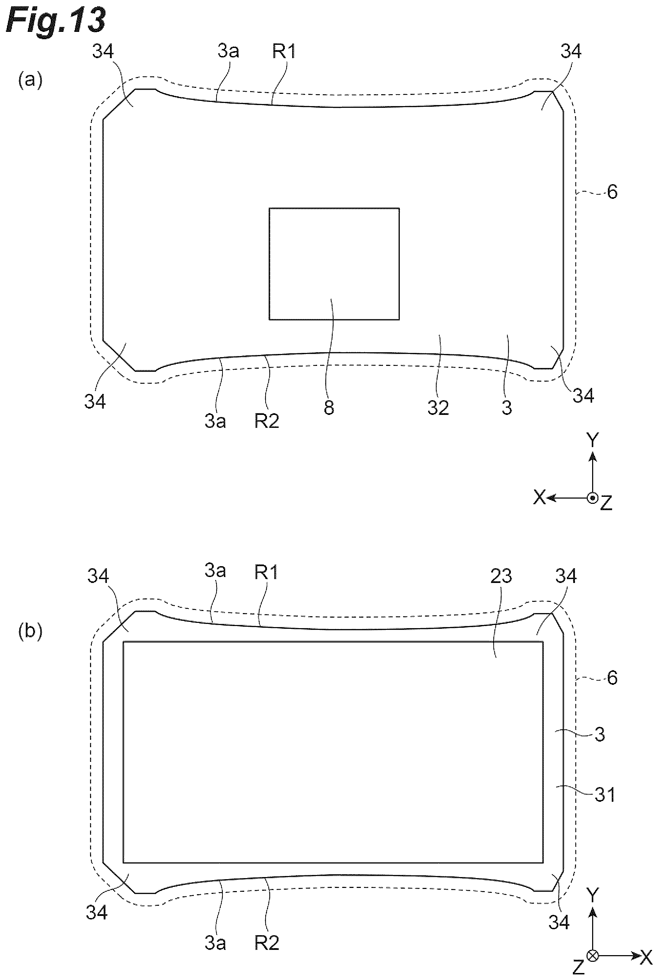

FIG. 13 illustrates a ninth modification. In the ninth modification, the second substrate 4 is not provided, and the battery 8 is provided on the second main surface 32 of the first substrate 3. FIG. 13(a) illustrates the second main surface 32 side of the first substrate 3, and FIG. 13(b) illustrates the first main surface 31 side of the first substrate 3. In the ninth modification, a first curved portion R1 (rounded portion) and a second curved portion R2 (rounded portion) curved so as to be recessed toward the central portion side of the first substrate 3 are formed on the pair of long sides 3a of the first substrate 3. As a result, the first substrate 3 is constricted when viewed from the Z direction. That is, the length of the first substrate 3 in the Y direction (the direction in which the pair of long sides 3a face each other) decreases from both ends of the long side 3a in the X direction (the extending direction of the pair of long sides 3a) toward the central portion. The readout substrate 23 is disposed on the first main surface 31 of the first substrate 3 so that the entire outer edge thereof is located inside the outer edge of the first substrate 3. Furthermore, four corner portions 34 of the first substrate 3 are chamfered (C-chamfered). The housing 6 is formed in a shape corresponding to the first substrate 3. That is, the housing 6 is formed in a shape having a curved portion corresponding to the first curved portion R1 and the second curved portion R2.

That is, the intraoral image capturing device of the ninth modification is "an intraoral image capturing device 1 including: the radiation detection unit 2 that detects radiation; the first substrate 3 on which the communication device 71 capable of wirelessly communicating with the outside and the battery 8 are provided; and the housing 6 that houses the radiation detection unit 2 and the first substrate 3, in which a curved portion recessed toward a center side of the first substrate 3 is formed on one edge of the first substrate 3, and the housing 6 is formed in a shape corresponding to the first substrate 3". According to this intraoral image capturing device, since the user's finger easily fits the housing 6, it is possible to prevent the user from dropping the intraoral image capturing device 1. Furthermore, since the four corner portions 34 of the first substrate 3 are chamfered, the readout substrate 23 can be protected from the impact applied to the first substrate 3.

The present disclosure is not limited to the above embodiment. For example, the material and shape of each configuration are not limited to the material and shape described above, and various materials and shapes can be adopted.

In the above embodiment, the connection member 5 electrically and physically connects the second substrate 4 to the first substrate 3, but the configuration of the connection member 5 is not limited thereto. For example, the connection member 5 may not electrically connect the first substrate 3 and the second substrate 4, while the connection member 5 physically connects the first substrate 3 and the second substrate 4. In this case, a member (for example, a wire or a cable) that electrically connects the first substrate 3 and the second substrate 4 may be provided separately from the connection member 5. Alternatively, the connection member 5 may not physically connect the first substrate 3 and the second substrate 4 (not used to fix the second substrate 4 to the first substrate 3), while the connection member 5 electrically connects the first substrate 3 and the second substrate 4. In this case, a member (for example, a cushion member or a pole member) that physically connects (fixes) the first substrate 3 and the second substrate 4 may be provided separately from the connection member 5. The intraoral image capturing device 1 may include a connection member (for example, a wire or a cable) that electrically connects the second substrate 4 to the first substrate 3 and a connection member (for example, a cushion member or a pole member) that physically connects the second substrate 4 to the first substrate 3.

In the above embodiment and modifications, the first cushion member 12 may be configured by a part of the housing 6. That is, instead of disposing the first cushion member 12, the housing 6 may have a cushion portion formed by forming the inner portion of the housing 6 to be softer than the outer portion of the housing 6. For example, the housing 6 may have a two-layer structure including a first layer located outside and a second layer (a cushion portion) located inside. The second layer is softer than the first layer. The material of the first layer (the outer portion of the housing 6) is, for example, nylon or ABS resin. The material of the second layer (the inner portion of the housing 6) is, for example, a material having a Young's modulus smaller than that of the material of the first layer. In this case, the impact acting on the housing 6 can be absorbed by the cushion portion, and the strength against the impact can be further enhanced. Similarly, the third cushion member 14 may be configured by a part of the housing 6.

In the above embodiment, the battery control unit that controls the battery 8 is provided on the first substrate 3, but the battery control unit may be provided on the second substrate 4. In this case, since the battery control unit is provided on the second substrate 4, it is possible to increase the mounting area of other elements on the first substrate 3.

The second substrate 4 may be formed of a hard substrate, or may be formed of a reinforcing plate and a flexible substrate provided on the reinforcing plate. The flexible substrate is, for example, a rectangular sheet-shaped FPC. The second substrate 4 may be directly disposed on the second main surface 32 of the first substrate 3. That is, the second substrate 4 may not be separated from the first substrate 3 in the Z direction. The disposition of the electronic components 72 is not limited to the example described above. For example, the electronic component 72 may not be disposed between the first substrate 3 and the second substrate 4.

In the above embodiment, the connection member 5 is connected to the second substrate 4 via the connector 9, but the connection member 5 may be connected to the first substrate 3 via the connector 9. In this case, the connection member 5 can be attachable to and detachable from the first substrate 3. The connector 9 may be omitted, and the connection member 5 may be not attachable to and detachable from the first substrate 3 and the second substrate 4. In this case, the height can be reduced as compared with the case of being connected by the connector 9, and it is also possible to increase the area in which the electronic component can be mounted in the first substrate 3 and the second substrate 4 as the connector 9 is not provided. In the above embodiment, the connection member 5 is not limited to the FPC, and may be a cable or the like. The battery 8 is not limited to a film-shaped battery.

In the above embodiment, the entire communication device 71 is located outside the outer edge of the battery 8 when viewed from the Z direction. However, only a part of the communication device 71 may be located outside the outer edge of the battery 8 when viewed from the Z direction, and the remaining part of the communication device 71 may be located inside the outer edge of the battery 8. Also in this case, it is possible to prevent the battery 8 from affecting the operation of the communication device 71 to some extent.

In the above embodiment, in the first substrate 3, a pair of corners located on one side in the X direction is chamfered (C-chamfered), but all four corners may be chamfered.

In the above embodiment, the scintillator 21 is in contact with the bottom wall portion 63 of the first cover 61, but the present invention is not limited thereto. For example, another member such as a cushion member may be provided between the scintillator 21 and the bottom wall portion 63, and the scintillator 21 may be in contact with the bottom wall portion 63 via the other member.

### Reference Signs List

: 1: Intraoral image capturing device, 2: Radiation detection unit, 3: First substrate, 32: Second main surface (main surface), 33: Side surface, 4: Second substrate, 41: Third main surface (surface), 5, 5B: Connection member, 6: Housing, 63: Bottom wall portion (first wall portion), 64: Side wall portion, 65: Bottom wall portion (second wall portion), 65a: Outer surface, 66: Side wall portion, 71: Communication device, 72: Electronic component, 8: Battery, 9: Connector, 10: Cushion member, 11: Main surface cushion member, 12: First cushion member, 13: Second cushion member, 14: Third cushion member, 15: Side surface cushion member.

## Claims

1. An intraoral image capturing device comprising:
a radiation detection unit that detects radiation;
a first substrate provided with a communication device capable of wirelessly communicating with an outside;
a second substrate provided with a battery and disposed on a side opposite to the radiation detection unit with respect to the first substrate;
a connection member that connects the second substrate to the first substrate; and
a housing that houses the radiation detection unit, the first substrate, the second substrate, and the connection member, wherein
an entire outer edge of the second substrate is located inside an outer edge of the first substrate when viewed from a thickness direction of the first substrate.

2. The intraoral image capturing device according to claim 1, wherein the second substrate is separated from the first substrate in a thickness direction of the first substrate.

3. The intraoral image capturing device according to claim 2, wherein
the first substrate is further provided with an electronic component, and
the electronic component is disposed between the first substrate and the second substrate.

4. The intraoral image capturing device according to any one of claims 1 to 3, wherein the connection member has flexibility.

5. The intraoral image capturing device according to any one of claims 1 to 4, wherein the connection member is connected to the first substrate or the second substrate via a connector, and is attachable to and detachable from the first substrate or the second substrate.

6. The intraoral image capturing device according to any one of claims 1 to 5, wherein the connection member includes a curved portion that is curved so as to protrude in a direction parallel to a main surface of the first substrate.

7. The intraoral image capturing device according to any one of claims 1 to 3, wherein the connection member is disposed between the first substrate and the second substrate and supports the second substrate.

8. The intraoral image capturing device according to claim 7, wherein the connection member is attachable to and detachable from at least one of the first substrate and the second substrate.

9. The intraoral image capturing device according to any one of claims 1 to 8, further comprising a cushion member disposed between the first substrate and the housing.

10. The intraoral image capturing device according to claim 9, wherein the cushion member includes a main surface cushion member disposed between a main surface of the first substrate and the housing.

11. The intraoral image capturing device according to claim 10, wherein the main surface cushion member includes a first cushion member disposed between the second substrate and the housing.

12. The intraoral image capturing device according to claim 10 or 11, wherein the main surface cushion member includes a second cushion member disposed between the first substrate and the second substrate.

13. The intraoral image capturing device according to any one of claims 10 to 12, wherein the main surface cushion member includes a third cushion member including a portion located inside an outer edge of the first substrate and outside an outer edge of the second substrate when viewed from a thickness direction of the first substrate.

14. The intraoral image capturing device according to any one of claims 9 to 13, wherein the cushion member includes a side surface cushion member disposed between a side surface of the first substrate and the housing.

15. The intraoral image capturing device according to any one of claims 1 to 14, wherein
the housing includes a cushion portion formed by forming an inner portion of the housing to be softer than an outer portion of the housing, and
the cushion portion is in contact with at least one of the first substrate and the second substrate.

16. The intraoral image capturing device according to any one of claims 1 to 15, wherein a size of the battery is 5% or more of a size of the first substrate when viewed from a thickness direction of the first substrate.

17. The intraoral image capturing device according to any one of claims 1 to 16, wherein the battery is provided on a surface of the second substrate, the surface being on a side opposite to the first substrate side.

18. The intraoral image capturing device according to any one of claims 1 to 16, wherein the battery is provided on a surface of the second substrate, the surface being on a side of the first substrate side.

19. The intraoral image capturing device according to any one of claims 1 to 18, wherein
the housing includes a first wall portion disposed on a side opposite to the second substrate with respect to the first substrate, a second wall portion disposed on a side opposite to the first substrate with respect to the second substrate, and a side wall portion connected to the first wall portion and the second wall portion, and
an outer surface of the second wall portion is formed flat over the entire surface.

20. The intraoral image capturing device according to any one of claims 1 to 19, wherein the battery is a film-shaped battery.

21. The intraoral image capturing device according to any one of claims 1 to 20, wherein the second substrate is further provided with a battery control unit that controls the battery.

22. The intraoral image capturing device according to any one of claims 1 to 21, wherein at least a part of the communication device is located outside an outer edge of the battery when viewed from a thickness direction of the first substrate.

23. The intraoral image capturing device according to any one of claims 1 to 22, wherein the entire communication device is located outside an outer edge of the battery when viewed from a thickness direction of the first substrate.
